(19) Europäisches Patentamt
European Patent Office
Office européen des brevets

(11) **EP 2 805 725 B1**

(12) **EUROPEAN PATENT SPECIFICATION**

(45) Date of publication and mention
of the grant of the patent:
**15.01.2020 Bulletin 2020/03**

(51) Int Cl.:
***A61K 36/28*** (2006.01)      ***A61P 35/00*** (2006.01)
***A61P 31/12*** (2006.01)

(21) Application number: **14176106.4**

(22) Date of filing: **12.05.2009**

(54) **Plant extract and its therapeutic use**

Pflanzenextrakt und seine therapeutische Verwendung

Extrait végétal et son utilisation thérapeutique

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **16.05.2008 GB 0808974**
**19.12.2008 CH 19982008**

(43) Date of publication of application:
**26.11.2014 Bulletin 2014/48**

(62) Document number(s) of the earlier application(s) in
accordance with Art. 76 EPC:
**09746155.2 / 2 288 363**

(73) Proprietor: **Viridis Pharmaceutical Limited
Road Town Tortola (VG)**

(72) Inventor: **Kreuter, Matthias-Heinrich
8880 Walenstadt (CH)**

(74) Representative: **Riederer Hasler & Partner
Patentanwälte AG
Kappelestrasse 15
9492 Eschen (LI)**

(56) References cited:
EP-A1- 0 496 230          WO-A-2007/057651
WO-A-2008/146009      DE-A1- 4 131 313
US-B1- 6 300 370

• **CERNAJ P ET AL: "TERPENOID COMPOUNDS
FROM DIFFERENT PARTS OF
ACHILLEA-COLLINA INFLORESCENCES",
BIOLOGIA PLANTARUM (PRAGUE), vol. 25, no.
3, 1983, pages 221-224, XP002730540, ISSN:
0006-3134**
• **LOGGIA R D ET AL: "Depressive effects of
Chamomilla recutita (L.) Rausch, tubularflowers,
on central nervous system in mice",
PHARMACOLOGICAL RESEARCH
COMMUNICATIONS, ITALIAN
PHARMACOLOGICAL SOCIETY, IT, vol. 14, no. 2,
1 February 1982 (1982-02-01), pages 153-162,
XP009180491, ISSN: 0031-6989**

**Description**

Field of the Invention

This invention relates to a plant extract and to its therapeutic use.

Background of the Invention

WO 03/101479 A1 describes the valuable therapeutic properties of a composition comprising various components, typically administered together by intramuscular injection. The composition that was used in the examples comprises a camomile extract, although no direct therapeutic activity is ascribed to it; rather, it is described as an anti-irritant whose presence may alleviate the unpleasant effects of the injection per se.

WO 2007/057651 A1 discloses a process for the removal of water-soluble contaminants having lipid groups, especially endotoxins from aqueous compositions of camomile.

In WO 01/13929 A1 is described a substantially pure biologically active extract isolated from Achillea millefolium. This extract, preferably a methanolic extract, has antineoplastic activity, and is used as anti-cancer agent.

P. Černaj et al. in Biologia Plantarum, May 1983, vol. 25, no. 3, 1983, pages 221-224 disclose a gas-liquid chromatography analysis of different parts of the inflorescence of Achillea millefolium. The extraction was carried out with n-hexane. The presence of terpenoids was demonstrated and differences in composition between extracts from different inflorescence parts were found.

In WO 2008/146009 A1 are described the effects of camomile essential oil or the seed oil of black cumin or a mixture of both oils on 5-lipoxygenase activity in the human granulocyte cell line HL 60 and on the DNA-synthesis in the human glioblastoma cell line U87MG. In this reference are also described the effects of said oils on the IL-6 release in the human macrophage cell line THP1 and on the DNA-synthesis in the human prostate cancer cell DU 145.

R. D. Loggia et al. in Pharmacological Research Communications, vol. 14, no. 2, 1982, pages 153-162 report depressive effects of an aqueous extract of Chamomilla recutita (L.) Rausch, tubular flowers, on central nervous system in mice.

In US 6 300 370 B1 is described a process for manufacturing essential camomile oil. In this process a camomile extraction residue is subjected to a steam distillation or to an aqueous distillation. Said camomile extraction residue is obtained by the treatment of camomile flowers and stalks with an aqueous or organic solvent or a mixture thereof, with or without a preceding steam distillation of the starting material.

P.Vilagines, P. Delaveau and R. Vilagines "Inhibition de la replication du poliovirus par un extrait de Matricaria chamomilla (L.)", Comptes Rendus de l'Academie des Sciences, Serie III, Tome 301, No. 6, 1985, pages 289 to 294 describe the effect of a hydroalcoholic extract of *Matricaria chamomilla L.* on the growth of poliovirus type 1. When this camomile extract is added during the early stage of poliovirus development, then said extract inhibits cellular and viral RNA synthesis. This inhibition is partially reversible.

Objects of the Invention

It is an object of the present invention to provide a composition for the treatment of an abnormal proliferative and/or viral condition.

It is a further object of the present invention to provide a composition for the synchronization and the S-phase arrest of abnormal proliferative mammalian cells, especially of cancer cells, in the human or animal body.

This synchronization shall comprise the induction of ornithine decarboxylase and/or the inhibition of topoisomerase II.

It is also an object of the present invention to provide a composition for the treatment of an abnormal proliferative condition wherein the treatment comprises the simultaneous or sequential administration of this composition and of at least one antitumor agent.

These objects are attained with the present invention.

Summary of the Invention

The invention is characterized by the characteristics as defined in the independent claims.

Preferred embodiments are defined in the dependent claims.

This composition may also consist of an organic extract of at least one *Chamomilla* plant and/or of at least one *Achillea* plant and of at least one anti-tumor agent, and occasionally of at least one pharmaceutical and/or dermatological acceptable auxiliary agent.

Surprisingly, it has now been found that a camomile extract, obtained from the flower heads, such as that described in WO 2007/057651 A1, has valuable therapeutic properties. In particular, it has been found that it can reduce the DNA- and RNA-synthesis without substantial effect on protein synthesis, from which utility in the treatment of cancer may be deduced.

Even more surprisingly it has been found that organic extracts of the tubular flowers of *Matricaria recutita L.* (Flores tubiformis) are suitable for the synchronization and the S-phase arrest of abnormal proliferative mammalian cells, especially of cancer cells.

This synchronization takes place due to the induction of ornithine decarboxylase (transfer from $G_0$-phase into $G_1$-phase) and the inhibition of topoisomerase II (accumulation and arrest in the early S-phase).

It was also found that the inhibition of topoisomerase II was more than 100-fold stronger with an organic extract than with an aqueous extract (with respect to the concentration for complete inhibition of the enzyme).

Due to the fact that the inhibition of the topoisomerase II is crucial for the effectiveness of cell synchronization the organic extracts of the tubular flowers of *Matricaria recutita L.,* (Flores tubiformis) of the present invention are much more potent than the corresponding aqueous extracts.

Description of the Invention

The invention is based on data obtained using an aqueous extract of camomile and on data obtained using organic extracts, especially alcoholic extracts, for example ethanolic extracts, of the tubular flowers of *Matricaria recutita L.,* (Flores tubiformis). Aqueous extracts serve as comparative examples only.

The available evidences show that the aqueous camomile extract obtained as described below maintains proteinbiosynthesis, whereas DNA- and RNA-biosynthesis is reduced. This is a good measure of the desirable properties of this extract.

The aqueous and/or organic extracts may be obtained by any suitable procedure known to those of ordinary skill in the art. The extracts may be obtained by using an aqueous and/or organic medium, especially an alcoholic medium, such as an ethanolic medium, and separated from other components.

A preferred procedure for the preparation of an aqueous extract is described in WO 2007/057651 A1.

Preferably, the composition according to the present invention additionally comprises at least one component selected from the group consisting of pharmaceutical aids, preferably selected from the group consisting of pharmaceutical agents and pharmaceutical excipients.

The aqueous extract may be subjected to a purification process. Such an extract comprises a multi-component mixture of water-soluble components. It may be obtained by adding water to the appropriate plant part, to obtain a suspension that is then usually heated to a temperature below the boiling point of water, e.g. 90-94°C, and then cooled to room temperature.

The aqueous extract is then subjected to two filtration steps. For the purposes of illustration only, these will be described below as microfiltration and ultrafiltration, respectively. Other techniques, such as use of a lipophilic barrier, may be suitable. Each filtration step may be conducted in one, two or more than two stages, if desired.

Microfiltration is applied in order to remove material that would otherwise compromise the effectiveness of the ultrafiltration step.

In the following part are described possible embodiments of the present invention. The following examples illustrate the present invention.

The analysis of bacterial endotoxins of the samples obtained in the Examples was performed with the Cambrex PyroGene assay using a dilution factor of 1:10.000.

**Examples 1 and 2** (comparative example)

45 g of yellow tubular camomile flowers (Chamomilla recutita) were mixed with 900 g of water (Aqua purificata, Ph. Helv.). This mixture was heated to a temperature between 90°C and 94°C within 20 to 30 minutes. Thereafter the mixture was stored at room temperature (15°C to 25°C) until a temperature between 30°C and 35°C was reached.

The drug residue was removed by deep layer filtration. The obtained crude filtrate was clarified by filtration through a 0.22 $\mu$m membrane.

To the clarified filtrate, 0.3% (Example 1) or 0.1% (Example 2), with respect to the extract mass, of ricinus oil (Ph.Eur.) was added. The obtained mixture was homogenized for 5 minutes. This so prepared extract was filtered (in tangential flow mode) with retentate recovery via a 0.22 $\mu$m membrane.

The obtained permeate was filtered (in tangential flow mode) with retentate recovery via a 0.1 $\mu$m membrane. Finally, the obtained permeate was filtered (in tangential flow mode) with retentate recovery via a 1000 kDa membrane. Residue of bacterial endotoxins in each final filtrate: < 100 EU/ml.

**Examples 3 to 5** (comparative example)

Example 1 was repeated, except that, instead of ricinus oil, 0.3% (Example 3), 1.0% (Example 4; VIP-E_Matr'06_1003) and 3.0% (Example 5), with respect to the extract mass, of mygliol (Ph. Eur.) was added to the clarified filtrate.

Residue of bacterial endotoxins in each final filtrate: < 100 EU/ml.

**Example 6** (comparative example)

This Example uses a revised protocol, in which heating and cooling were performed, not in an autoclave but in a 10 L double layer vessel under stirring (max. temperature of heating device 140°C).

Mygliol was added instead of ricinus oil. The mygliol was "Mygliol 812 for parenteral use" from Hänseler. The mixture was stirred at room temperature for 10 minutes, instead of homogenization.

Microfiltrations according to the above described process were all performed with Millipore Pellicon 2 systems. For better practicability and to avoid time-consuming cleaning procedures, the microfiltrations in this Example were performed with the following equipment:

| Filtration | Filter System | Cartouche |
|---|---|---|
| 0.2 $\mu$m filtration | Millipore Pellicon 2 | Durapore 0.2 $\mu$, C-screen |
| 0.1 $\mu$m filtration | One way filter | Millipack 200, 0.1 $\mu$m |
| 1000 kDa filtration | Millipore Pellicon 2 | Biomax 1000 kDa, V-Screen |
| 0.2 $\mu$m filtration | One way filter | Millipack 200, 0.2 $\mu$m |

In addition, phenol was added, for stabilization of the extract. The amount of added phenol was 6.0-8.0 mg/ml. It was added after the 1000 kDa filtration. After the addition, the suspension was stirred for approximately 10 minutes, until all phenol was dissolved.

The endotoxin level was low in each case.

**Example 7** (Preparation of a liquid extract ViP-E_Matr'08_1102)

100 g of tubular flowers from the inflorescence of *Matricaria recutita L.* were extracted under stirring at a temperature between 40°C and 60°C during 2 hours with 500 g 80% (m/m) ethanol, corresponding to a drug to solvent ratio of 1/5. Subsequently the preparation was subjected to a deep layer filtration using a cellulose filter (AF 6 Filtrox®). 385 g of a clear dark brown liquid extract with a solid content of 4.43 % (m/m) were obtained.

**Example 8** (Preparation of a liquid extract ViP-E_Matr'08_1106)

100 g of tubular flowers from the inflorescence of *Matricaria recutita L.* were extracted under stirring at a temperature between 40°C and 60°C during 2 hours with 500 g 90% (m/m) ethanol, corresponding to a drug to solvent ratio of 1/5. Subsequently the preparation was subjected to a deep layer filtration using a cellulose filter (AF 6 Filtrox®). 398 g of a clear dark brown liquid extract with a solid content of 1.89 % (m/m) were obtained.

**Example 9** (Preparation of a liquid extract ViP-E_Matr'08_1105)

100 g of tubular flowers from the inflorescence of *Matricaria recutita L.* were extracted under stirring at a temperature between 40°C and 60°C during 2 hours with 500 g 99.9% (m/m) ethanol, corresponding to a drug to solvent ratio of 1/5. Subsequently the preparation was subjected to a deep layer filtration using a cellulose filter (AF 6 Filtrox®). 412 g of a clear dark brown liquid extract with a solid content of 1.54 % (m/m) were obtained.

The following examples illustrate the pharmacological activity profile of the extracts of examples 4, 7, 8 and 9.

**Example 10** (comparative example; Induction of ornithine decarboxylase expression)

With the liquid extract VIP-E_Matr'06_1003 prepared according to example 4 were carried out ornithine decarboxylase expression experiments.

For the measurement of the induction of ornithine decarboxylase expression this extract was added to HepG2 cells in concentrations of 150, 100, or 30 μg/ml. The so treated cells were cultivated during 6 hours, 24 hours, or 48 hours in 10% FBS culture medium.

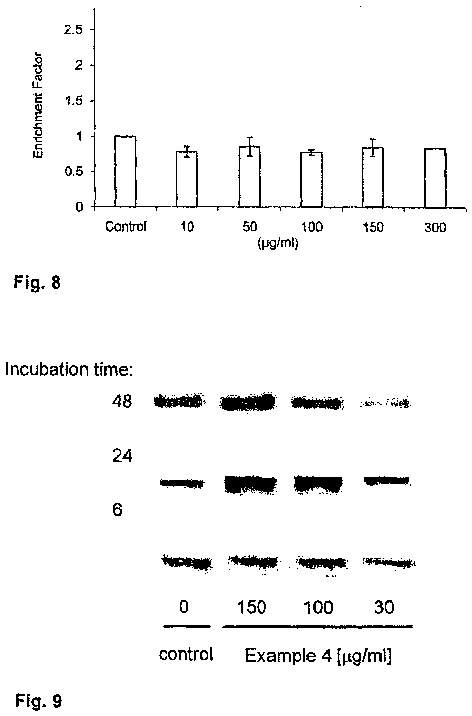

Then was determined the change of the amount of the ornithine decarboxylase by means of the Westernblot analysis. It is obvious from the data shown in Figure 9 that the extract induces the ornithine decarboxylase expression in a concentration-dependent manner.

**Example 11** (comparative example; Inhibition of topoisomerase I activity)

With the liquid extract ViP-E_Matr'08_1102 prepared according to example 7 were carried out topoisomerase I activity experiments with extract concentrations of 0.3, 1, 3, 10, or 30 μg/ml. As positive control camptothecin was carried along.

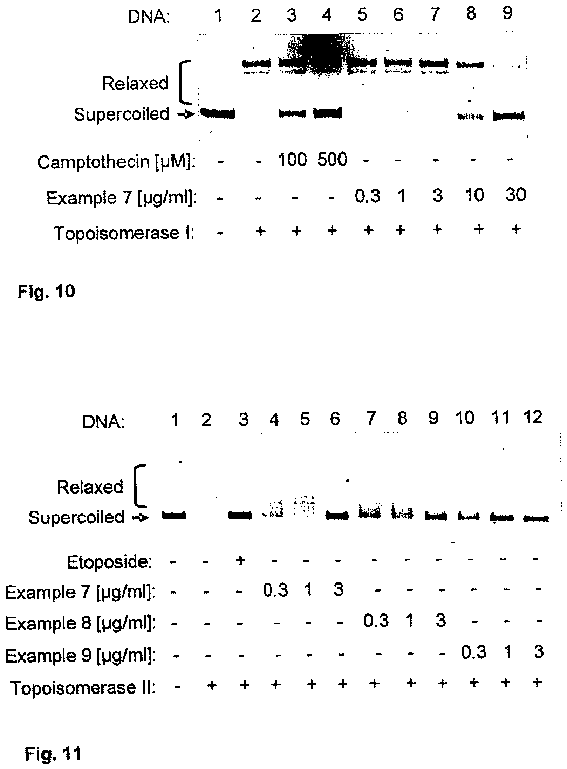

For the measurement of the inhibition of topoisomerase I activity this extract was added to purified human DNA topoisomerase I and the "Topoisomerase I Drug Screening Kit" from TopoGEN. Thereby it was proceeded according to the manufacturer's protocol. The DNA was separated by electrophoresis on an agarose gel and visualized under UV light after staining with ethidium bromide. With this approach the relaxed DNA (topoisomers) generated by topoisomerase I could be detected and clearly separated from the supercoiled DNA substrate. It is obvious from the data shown in Figure 10 that the extract inhibits topoisomerase I activity only very weak but in a concentration-dependent manner.

**Example 12** (Inhibition of topoisomerase II activity)

With the liquid extract ViP-E_Matr'08_1102, prepared according to example 7, the liquid extract ViP-E_Mtatr'08_1106, prepared according to example 8, or the liquid extract ViP-E_Matr'08_1105, prepared according to example 9, were carried out topoisomerase II activity experiments with extract concentrations of 0.3, 1, or 3 μg/ml. As positive control etoposide was carried along.

The inhibition of topoisomerase II was determined using purified human DNA topoisomerase II$\alpha$ and the "Topoisomerase II Drug Screening Kit" from TopoGEN.

Thereby it was proceeded according to the manufacturer's protocol. The DNA was separated by electrophoresis on an agarose gel and visualized under UV light after staining with ethidium bromide. With this approach the relaxed DNA (topoisomers) generated by topoisomerase II could be detected and clearly separated from the supercoiled DNA substrate.

It is obvious from the data shown in Figure 11 that all of the extracts according to the present invention strongly inhibit the topoisomerase II activity in a concentration-dependent manner. The extent of inhibition clearly increases with increasing concentrations of ethanol % m/m as extraction solvent (99 %>90%>80%). The extract prepared with 99 % m/m ethanol showed a practically complete inhibition of the enzyme activity even at a concentration as low as 300 ng/ml.

This inhibition was more than 100-fold stronger than the inhibition obtained with the aqueous extract of example 4. Nearly 150 μg/ml were necessary for complete inhibition (data not shown).

**Example 13** (comparative example; Induction of cell cycle arrest in S-phase)

With the liquid extract VIP-E_Matr'06_1003 prepared according to example 4 were carried out cell cycle analysis experiments. As positive control for cell cycle arrest in S-phase camptothecin was carried along.

For the measurement of the induction of cell cycle arrest this extract was added to HepG2 cells in concentrations of 10, 50, 100, or 150 μg/ml. The so treated cells were cultivated during 48 hours in 10% FBS culture medium.

Then was determined the change of the amount of cells in G1-, S- or G2-phase of the cell cycle by means of the cell cytometry analysis.

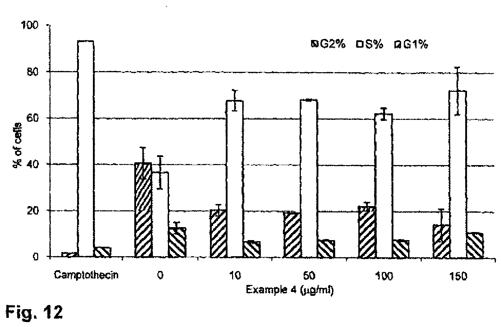

It is obvious from the data shown in Figure 12 that the extract induces a strong cell cycle arrest in S-phase already after 48 hours of incubation at concentrations as low as 10 μg/ml.

**Example 14** (Preparation of a resinous extract)

From a liquid extract obtained in analogy to example 9 the solvent was evaporated under reduced pressure (300 to 10 mbar) and at slightly elevated temperature (35°C to 45°C). 6.4 g of a dark brown resinous extract with a dry material content of >99 % (m/m) were obtained.

The obtained resinous extract was found to be free or nearly free of essential oils (< 0.01 % m/m). The content of ethanol was found to be < 0.05 % (m/m), and the content of water was found to be < 0.01% (m/m).

**Example 15** (Preparation of a water-free liquid extract)

To 6.4 g of a liquid extract obtained in analogy to example 9 were added 6.4 g of oleic acid (Ph.Eur.) and 19.2 g of Macrogol 400 (Ph.Eur.).

The ethanol was evaporated under reduced pressure (300 to 10 mbar) and at slightly elevated temperature (35°C to 45°C). 31.8 g of a dark brown free-flowing liquid extract with a content of non-volatile components of >99 % (m/m) were obtained.

The obtained water-free liquid extract was found to be free or nearly free of essential oils (< 0.01% m/m). The content of ethanol was found to be < 0.05 % (m/m), and the content of water was found to be < 0.01% (m/m).

**Example 16** (Preparation of a water-free liquid extract)

To 10 g of the resinous extract obtained in analogy to example 14 were added 10 g of oleic acid (Ph.Eur.) and 20 g of Macrogol 400 (Ph.Eur.).

This mixture was homogenized.

The so obtained water-free liquid extract had a content of non-volatile components of >99 % (m/m).

**Example 17** (comparative example)

20 g of yellow tubular camomile flowers (*Anthemis nobilis L.*) were mixed with 380 g of water (Aqua purificata, Ph. Helv.). This mixture was heated to a temperature between 90°C and 94°C within 20 to 30 minutes. Thereafter the mixture was stored at room temperature (22°C) until a temperature between 30°C and 35°C was reached.

The drug residue was removed by deep layer filtration. The obtained crude filtrate was clarified by filtration through a 0.22 $\mu$m membrane.

To the clarified filtrate, 0.3% mygliol (Ph. Eur.), with respect to the extract mass, was added. The obtained mixture was homogenized for 5 minutes. This so prepared extract was filtered (in tangential flow mode) with retentate recovery via a 0.22 $\mu$m membrane.

The obtained permeate was filtered (in tangential flow mode) with retentate recovery via a 0.1 $\mu$m membrane. Finally, the obtained permeate was filtered (in tangential flow mode) with retentate recovery via a 1000 kDa membrane.

There were obtained 309 g of a clear, aqueous extract with a dry material content of 1.1 % m/m.

**Example 18** (comparative example)

Same procedure as in Example 17 but 20 g of the whole flower heads of *Anthemis nobilis L* were used.

There were obtained 295 g of a clear, aqueous extract with a dry material content of 1.6 % m/m.

**Example 19** (comparative example)

40 g of yellow tubular flowers (*Achillea millefolium L.*) were mixed with 760 g of water (Aqua purificata, Ph. Helv.). This mixture was heated to a temperature between 90°C and 94°C within 20 to 30 minutes. Thereafter the mixture was stored at room temperature (22°C) until a temperature between 30°C and 35°C was reached.

The drug residue was removed by deep layer filtration. The obtained crude filtrate was clarified by filtration through a 0.22 $\mu$m membrane.

To the clarified filtrate, 0.3% mygliol (Ph. Eur.), with respect to the extract mass, was added. The obtained mixture was homogenized for 5 minutes. This so prepared extract was filtered (in tangential flow mode) with retentate recovery via a 0.22 $\mu$m membrane.

The obtained permeate was filtered (in tangential flow mode) with retentate recovery via a 0.1 $\mu$m membrane. Finally, the obtained permeate was filtered (in tangential flow mode) with retentate recovery via a 1000 kDa membrane.

There were obtained 667 g of a clear, aqueous extract with a dry material content of 1.3 % m/m.

**Example 20** (comparative example)

Same procedure as in Example 19 but 40 g of the whole flower heads of *Achillea millefolium L.*) were used.

There were obtained 634 g of a clear, aqueous extract with a dry material content of 1.4 % m/m.

**Example 21**

20 g of tubular flowers from the inflorescence of *Anthemis nobilis L.* were extracted under stirring at a temperature between 40°C and 60°C during 2 hours with 100 g 99.9% (m/m) ethanol, corresponding to a drug to solvent ratio of 1/5. Subsequently the preparation was subjected to a deep layer filtration using a cellulose filter (AF 6 Filtrox®). 71 g of a clear dark brown liquid extract with a solid content of 1.62 % (m/m) were obtained.

**Example 22**

40 g of tubular flowers from the inflorescence of *Achillea millefolium L,* were extracted under stirring at a temperature between 40°C and 60°C during 2 hours with 200 g 99.9% (m/m) ethanol, corresponding to a drug to solvent ratio of 1/5. Subsequently the preparation was subjected to a deep layer filtration using a cellulose filter (AF 6 Filtrox®). 153 g of a clear dark brown liquid extract with a solid content of 1.62 % (m/m) were obtained.

**Example 23** (DNA synthesis (BrdU Incorporation Assay)

The liquid extract according to Example 21 was examined on its ability to inhibit DNA synthesis in human hepatocellular carcinoma cells (HepG2) *in vitro.*

To determine cellular proliferation, freshly synthesized DNA was quantified using the 5-Bromo-2'-deoxy-uridine (BrdU) Labeling and Detection Kit III (Roche Applied Science; Mannheim, Germany). BrdU is a thymidine analog which would be incorporated into new cellular DNA. [See: Gratzner, H. G. (1982) Science 218, 474-475].

In short, human hepatocellular carcinoma cells (HepG2) were harvested by trypsinisation and seeded at 10'000 cells/well in 96 well micro-plates. After 24 hours of pre-incubation at 37°C and 5% $CO_2$, the cells were treated with the liquid extract according to Example 21 at the following concentrations for 30 hours: 0, 1, 3, 10 and 100 $\mu$g/ml.

10 $\mu$l of BrdU (100$\mu$M) were then added to the cells and further incubated for 18 hours. After incubation, the cells were washed 3 times with culture media to remove the excess BrdU before being fixed with ethanol. Prior to incubation with the anti-BrdU antibody, labeled with peroxidase (POD), DNA was partially digested with nucleases to allow the antibody to access the incorporated BrdU. After washing the excess antibody, the POD substrate ABTS was added. POD catalyzes the cleavage of ABTS, producing a coloured reaction product. The absorbance of the reaction product was measured at 405 nm (reference wavelength at 490 nm) with a Safire multifunctional microplate reader (Tecan, Mannedorf, Switzerland). The measured absorbance per well correlates directly to the level of BrdU incorporated into cellular DNA.

Sample points were measured as triplicates; errors were expressed as standard deviation (data no shown).

The data of the samples were expressed as a percentage of the solvent control values, and the $IC_{50}$ values were calculated using GraphPad Software Inc (Prism, version 5, San Diego, CA. USA).

Results

The results showed that the liquid extract according to Example 21 had an $IC_{50}$ value of approximately 7$\mu$glml (6.228 to 7.881 $\mu$g/ml). Thus, said liquid extract inhibited the growth of human hepatocellular carcinoma cells (HepG2) in a dose dependent manner.

Compositions according to the invention can be formulated by methods known to those skilled in the art. Pharmaceutically acceptable components should be used.

Administration is preferably by intravenous or, more preferably, intramuscular injection.

The pharmaceutical composition containing the active ingredient may be also in a form suitable for oral use.

Therapeutic use involves the treatment (and possibly also the prevention) of cancer, for example lung cancer, liver cancer and cancer of the pancreas. Other uses include topical conditions such as psoriasis, scleroderma and pemphigus, infectious bronchitis, cancers including sarcomas (such as Kaposi's sarcoma), leukemia, skin cancer and the carcinomas whose treatment is specifically illustrated below, as well as AIDS. More generally, the composition may be used for therapy of proliferative and viral conditions, especially those associated with DNA- or RNA-viruses. The action on RNA-viruses may be direct, while the action on DNA-viruses and cancer at least may be progressive. The medicament may also be useful in therapy of other genetic disorders such as motor neurone diseases and multiple sclerosis.

The medicament can also be used to treat other viral conditions. For example, the virus may be a coronavirus, as in the case of SARS (severe acute respiratory syndrome). Further, the medicament may have utility in veterinary medicine, e.g. in fowl's diseases such as Newcastle disease and fowl pox.

The following study illustrates further aspects obtained with the extract according to example 4 (a comparative example).

In the following part is given a short description of the Figures:

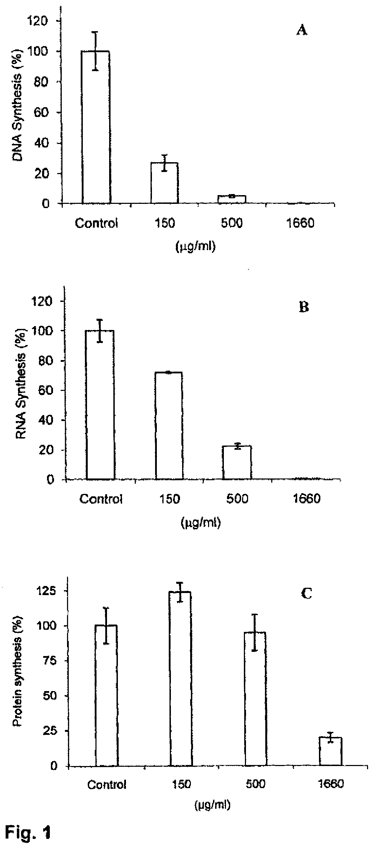

Figure 1 relates to System 4 and shows the effect of the extract according to example 4 on the DNA-synthesis (A), on the RNA-synthesis (B), and on the proteinbiosynthesis (C) in HepG2 cells. The used extract concentrations are 150, 500, and 1660 µg/ml.

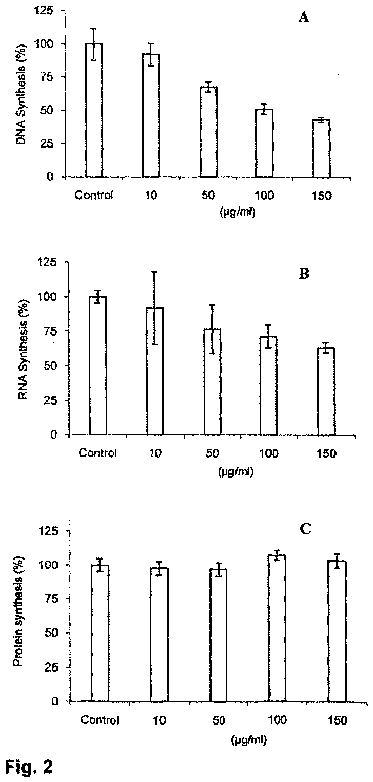

Figure 2 is in analogy to Figure 1, but the used extract concentrations are 10, 50, 100, and 150 µg/ml.

Figure 3 relates to System 4 and shows the effect of the extract according to example 4 on RNA-synthesis (A) and proteinbiosynthesis (B), calculated in consideration of the results shown in Figure 2(A).

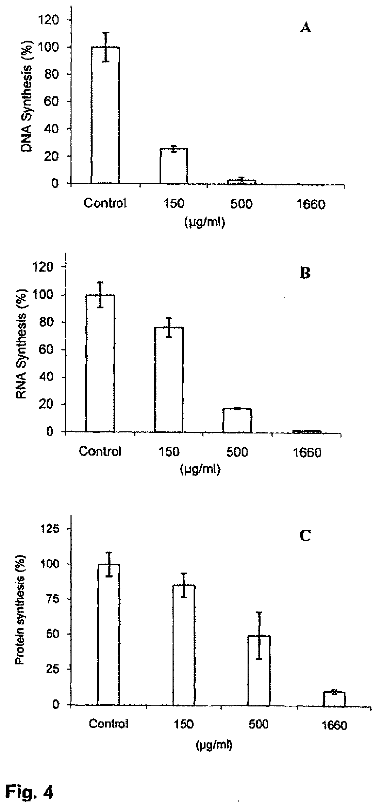

Figure 4 relates to System 4 and shows the effect of the extract according to example 4 on the DNA-synthesis (A), on the RNA-synthesis (B), and on the proteinbiosynthesis (C) in HT1376 cells. The used extract concentrations are 150, 500, and 1660 µg/ml.

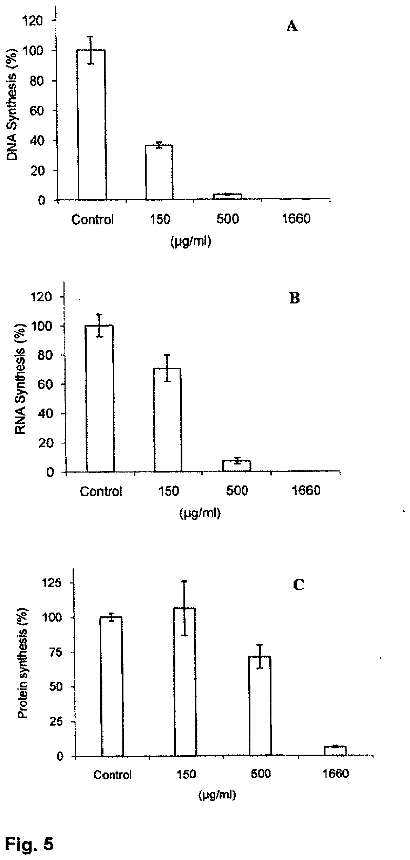

Figure 5 relates to System 4 and shows the effect of the extract according to example 4 on the DNA-synthesis (A), on the RNA-synthesis (B), and on the proteinbiosynthesis (C) in C33-A cells. The used extract concentrations are 150, 500, and 1660 µg/ml.

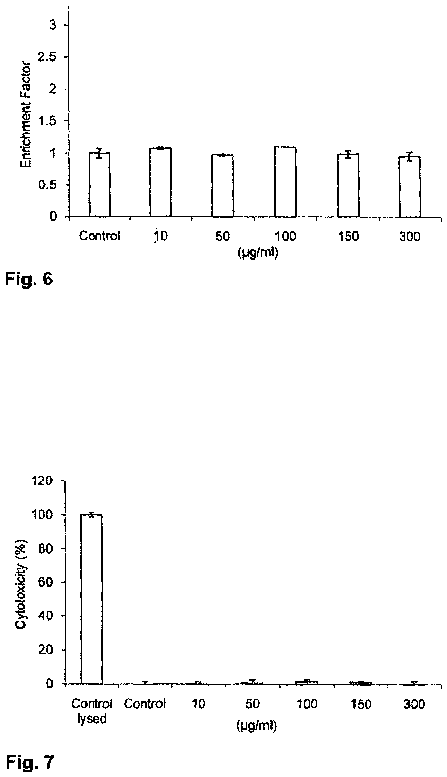

Figure 6 relates to System 5 and shows the effect of the extract according to example 4 on the induction of the apoptosis in HepG2 cells after 24 hours. The used extract concentrations are 10, 50, 100, 150, and 300 µg/ml.

Figure 7 relates to System 5 and shows the effect of the extract according to example 4 on the cytotoxicity (membrane integrity) in HepG2 cells after 24 hours. The used extract concentrations are 10, 50, 100, 150, and 300 µg/ml.

Figure 8 relates to System 5 and shows the effect of the extract according to example 4 on the induction of the apoptosis in HepG2 cells after 48 hours. The used extract concentrations are 10, 50, 100, 150, and 300 µg/ml.

Figure 9 relates to example 10 and shows the effect of the extract according to example 4 on the induction of the ornithine decarboxylase expression in HepG2 cells after 6, 24, and 48 hours. The used extract concentrations are 30, 100, and 150 µg/ml.

Figure 10 relates to example 11 and shows the effect of the extract according to example 7 on the inhibition of topoisomerase I in a cell-free assay. The used extract concentrations are 0.3, 1, 3, 10, and 30 µg/ml. As positive control camptothecin was used.

Figure 11 relates to example 12 and shows the effect of the extracts according to examples 7, 8, and 9 on the inhibition of topoisomerase II in a cell-free assay. The used extract concentrations are 0.3, 1, and 3 µg/ml. As positive control etoposide was used.

Figure 12 relates to example 13 and shows the effect of the extract according to example 4 on the induction of cell cycle arrest in S-phase in HepG2 cells. The used extract concentrations are 10, 50, 100, and 150 µg/ml. As positive control camptothecin was used.

Figure 13 relates to example 12 and shows the effect of the extract according to example 7 on the inhibition of topoisomerase II in a cell-free assay. The used extract concentrations are 0.3, 1, 3, 10, and 30 µg/ml. As positive control etoposide was used. For comparison reasons 0.3, 3, and 30 µg/ml of α-bisabofol or 0.3, 3, and 30 µg/ml of the essential oil of *Matricaria recutita L.* was used.

1) Aim of the study

System 4: RNA-, DNA- and Proteinbiosynthesis

The influence of the extract according to example 4 on RNA-, DNA- and proteinbiosynthesis using three different cell lines (HepG2: liver carcinoma cells; C33-A: cervix carcinoma cells and HT1376: bladder carcinoma cells) was examined *in vitro*.

System 5: Apoptosis and Membrane Integrity

Additionally the influence of the extract according to example 4 on the induction of apoptosis was tested on HepG2 cells. At the same time the cytotoxicity (membrane integrity) of the extract according to example 4 was examined on HepG2 cells.

**2) Material and Methods**

*2. 1) Samples and sample preparation*

| Extract | Description | Origin | Lot | Extract Type |
|---------|-------------|--------|-----|--------------|

(continued)

| Extract | Description | Origin | Lot | Extract Type |
|---|---|---|---|---|
| VIP-E_ Matr'06_1003 | *Matricaria recutita* tubular flowers | Pentapharm | 578-01/End; 7.20.2006 | Aqueous |
| VIP-E_Matr'06_1003 was prepared according to example 4. | | | | |

*2.2) Assay conditions*

| Assay | Cell lines | Incubation time | Sample | Sample concentration |
|---|---|---|---|---|
| RNA-Synthesis | HepG2C33-A HT1376 | 24h | VIP-E_ Matr'06_1003 | 150 / 500 / 1660µg/ml 10 / 50 / 100 / 150µg/ml |
| DNA-Synthesis | HepG2C33-A HT1376 | 24h | VIP-E_ Matr'06_1003 | 150 / 500 / 1660µg/ml |
| Proteinbiosynthesis | HepG2C33-A HT1376 | 24h | VIP-E_ Matr'06_1003 | 150 / 500 / 1660µg/ml |
| Apoptosis | HepG2 | 24h | VIP-E_ Matr'06_1003 | 10 / 50 / 100 / 150 / 300µg/ml |
| Apoptosis | HepG2 | 48h | VIP-E_ Matr'06_1003 | 10 / 50 / 100 / 150 / 300µg/ml |
| Cytotoxicity (membrane integrity) | HepG2 | 24h | VIP-E_ Matr'06_1003 | 10, 50, 100, 150, 300µg/ml |

*2.3) Assays*

2.3.3) RNA-/DNA-Synthesis

For the RNA- and DNA-synthesis, assay cells (HepG2: hepatocellular carcinoma, human; C33-A: cervical carcinoma, human and HT1376: bladder carcinoma, human) were harvested by trypsinisation and seeded at 10'000 cells/well in a 96 well plate. After treatment of the cells with the samples at the required concentrations the plate was incubated for 2 hours at 37°C and 5% $CO_2$. The cells were pulsed with 5-[3H]-Uridine (1µCi/ml) (Perkin Elmer) for the RNA-synthesis and with 6-[3H]-Thymidine (1µCi/ml) (Perkin Elmer) for the DNA-synthesis during a further incubation period of 24 hours, The cells were washed with PBS and fixed twice with methanol for 5 min. The protein was precipitated by 0.3N TCA. After a washing step 150µl 0,3N NaOH was added for 15min to lyse the cells. Negative controls t(0) were carried out with the samples without cells.

To detect the incorporated 5-[3H]-Uridine for the RNA-synthesis and the 6-[3H]-Thymidine for the DNA-synthesis the samples were transferred in scintillation tubes with scintillation cocktail. The quantification was performed in a Tri-Carb 1900 TR liquid scintillation counter (Packard, USA).

The effect of several concentrations of samples was measured by determining amount of radiolabel (dpm) under the assay conditions. Dose related values were expressed as a percentage of the positive control values. Sample points were measured as duplicates, errors are expressed as difference from the mean.

The specific cellular 5-[3H]-Uridine incorporation ratio (%) was calculated in consideration of the results from the DNA-synthesis assay. The respective synthesis values (%) were divided by the DNA-synthesis coefficient.

2.3.4) Proteinbiosynthesis

For the proteinbiosynthesis assay cells (HepG2: hepatocellular carcinoma, human; C33-A: cervical carcinoma, human and HT1376 : bladder carcinoma, human) were harvested by trypsinisation and seeded at 10'000cells/well in a 96 well plate. After treatment of the cells with the samples at the required concentrations the plate was incubated for 2 hours at 37°C and 5% $CO_2$. The cells were pulsed with L-[3,4,5-[3H(N)]]-Leucine, (1µCi/ml) (Perkin Elmer) during a further incubation period of 24 hours. The cells were washed twice with PBS, lysed with RIPA-buffer and incubated on ice for 15min. The lysate was transferred in a tube. 250μl ice cold TCA (20%) was added and the samples were incubated on ice for further 15min, After centrifugation for 20min at 10'000xg and 4°C the supernatant was removed and the pellet was resuspended in 250μl TCA (5%) and centrifuged again. The received lysates of each sample were resuspended in 250μl NaOH (0,2N) and heated for 2 min at 50°C. Negative controls t(0) were carried out with the samples without cells.

To detect the incorporated L-[3,4,5-3H(N)]-Leucine the samples were transferred in scintillation tubes with scintillation cocktail. The quantification was performed in a Tri-Carb 1900 TR liquid scintillation counter (Packard, USA).

The effect of several concentrations of the sample was measured by determining the amount of radiolabel (dpm) under the assay conditions. Dose related values were expressed as a percentage of the positive control values. Sample points were measured as quadruplicates, errors are expressed as standard deviations.

The specific cellular L-[3,4,5-3H(N)]-Leucine incorporation ratio (%) was calculated in consideration of the results from the DNA-synthesis assay, The respective synthesis values (%) were divided by the proliferation coefficient.

2.3.5) Apoptosis assay

HepG2 cells (hepatocellular carcinoma, human) were harvested by trypsinisation and seeded at 10'000 cells/well in 100μl in a 96 well plate. After 24h, 20μl samples and 80μl of fresh medium (total volume 200μl) were added. The plate was further incubated for 24 hours and in an additional experiment for 48hours. After the centrifugation of the plate for 10min at 200xg, the supernatant was discarded and the pellet was lysed for 30 min at room temperature in 200 μl Lysis buffer. The lysate was finally centrifuged for 10min at 200xg. The solvent controls (= negative controls) were performed with solvent instead of sample. Seeding and incubation were performed in an incubator at 37°C and 5% $CO_2$.

The cytoplasmatic histone associated DNA-fragments (mono- and oligonucleosomes) in lysate supernatant were determined *in vitro* with a Cell Death Detection ELISA[plus] Kit (Roche, Cat-No: 11 774 425 001) according to the supplier recommendation.

The absorbance was measured with a microplate reader device (TECAN Infinite M200) at a wavelength of $\lambda$ = 405nm and a reference wavelength of $\lambda$ = 490nm. The specific enrichment factor of mono- and oligonucleosomes released into the cytoplasm was calculated as following:

$$\text{Enrichment factor} = \text{absorbance sample/absorbance of the corresponding negative control}$$

Sample points were measured as duplicates, errors are expressed as difference from the mean.

2.3.6) LDH-Cytotoxicity assay (Induction of necrosis)

LDH-Cytotoxicity assay (membrane integrity/induction of necrosis): the cytotoxicity was tested on HepG2 cells (hepatocellular carcinoma, human). The cells were seeded in a 96 well plate at 10'000 cells per well over night. 20 μl of the respective samples and solvent controls at the indicated concentrations and 80 μl of fresh medium (total volume 200 μl) were added. Seeding and incubation were performed in an incubator at 37°C and 5% $CO_2$. After an incubation period of 24 hours, the membrane integrity was measured with a LDH-cytotoxicity Assay Kit (BioVision, #K311-400, CA USA).

The membrane integrity of the cells was determined by measuring the activity of cytosolic lactate dehydrogenase (LDH) released into the medium under the influence of samples. The activity of the enzyme was measured by determining the formazan produced from the tetrazolium salt INT as substrate. The quantity of formazan was measured directly by determining the optical density (OD) with a plate reader (TECAN Infinite M200) at a wavelength of $\lambda$=490 nm.

For each test concentration, the OD values of the background (assay mixture with samples but without cells) was subtracted from OD measurements with cells. $OD_{490}$-values were transformed into percentage values with cytotoxicity readings of 100% corresponding to measurements of the cells lysed (triton X-100) with solvent, and cytotoxicity readings of 0% corresponding to cells incubated with solvent only. The optical measurements were performed as duplicates. Errors were expressed as difference from the mean.

**4) Summary**

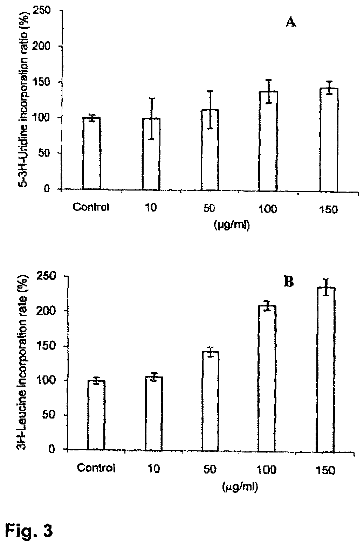

With respect to the DNA-, RNA- and proteinbiosynthesis the extract according to example 4 showed the following effects: the DNA-synthesis is dose dependent suppressed, while up to considerably high doses the RNA-synthesis is still intact and the proteinbiosynthesis is only marginal affected or, if adapted to the amount of proliferating cells, even increased (Figure 3). This effect is evident for all three cell lines in non-cytotoxic dosage ranges and can be interpreted as a sign for a cell arrest and a possible differentiation. All tested carcinoma cells, HepG2 hepatocarcinoma, HT1376 bladder carcinoma and C33A cervix carcinoma, showed this phenomenon.

In the subsequently initiated experiments with HepG2 cells for discrimination between apoptosis and necrosis (indicated by LDH-leakage and loss of cell membrane integrity), after 24 hours we detected no induction of apoptosis even in the highest tested dosage. The extract according to example 4 did not show any effects, neither apoptosis nor cytotoxicity.

Therefore it was decided to conduct an additional experiment with an incubation of the cells for 48 hours. Interestingly, after 48 hours it was found also no induction of apoptosis.

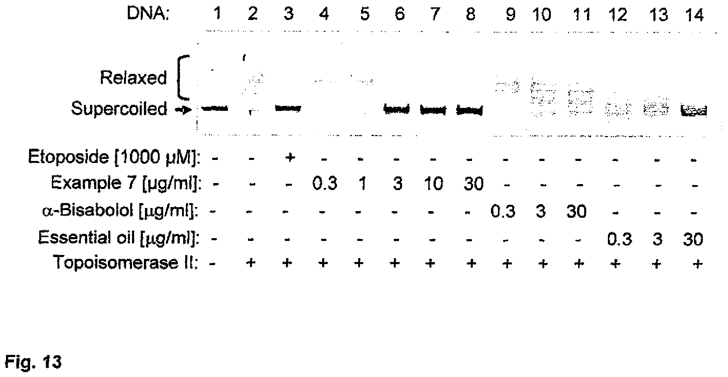

It is obvious from Figure 13 that the extract according to example 7 shows a total inhibition of topoisomerase II activity starting at a concentration of 3 $\mu$g/ml. Below the concentration of 3 $\mu$g/ml no inhibition is detectable.

$\alpha$-Bisabolol, one of the main components of the essential oil of a *Chamomilla* plant, did not influence the topoisomerase II activity in all tested concentrations.

The essential oil of *Matricaria recutita L.* hardly inhibited the topoisomerase II activity. Only an incomplete inhibition at a relatively high concentration of 30 $\mu$g/ml was observed.

For the purpose of the present invention the terms "containing" or "comprising" shall mean that also additional compounds or components may be present, whereas the term "consist" shall mean that no additional compounds or components than those explicitly mentioned are present.

Disclosed are inter alia:

1. A composition comprising an organic extract according to claim 1.

2. The composition according to 1, characterized in that said composition comprises occasionally at least one pharmaceutical and/or dermatological acceptable auxiliary agent.

3. The composition according to one of 1 to 2, characterized in that the treatment of the abnormal proliferative condition comprises the synchronization and the S-phase arrest of abnormal proliferative mammalian cells, especially of cancer cells.

4. The composition according to 3, characterized in that said synchronization comprises the induction of ornithine decarboxylase and/or the inhibition of topoisomerases, especially topoisomerase II.

5. The composition according to one of 1 to 4, characterized in that said organic extracts are obtainable by an extraction with an alcohol, especially an alcohol with 2 to 6 carbon atoms, whereby ethanol is especially preferred, or with a ketone especially a ketone with 3 to 5 carbon atoms, whereby acetone is especially preferred.

6. The composition according to one of 1 to 5, characterized in that the extracts are obtainable from the flower heads of the Chamomilla plant, especially Flores tubiformis that are free of apigenin and apigenin-glycosides, and/or from the herbs and/or from the flower heads of the Achillea plant, especially Flores tubiformis.

7. The composition according to one of 1 to 6, characterized in that the Chamomilla plant is selected from the group consisting of

- Matricaria species, especially
- Matricaria recutita L
- Matricaria discoidea DC.
- Anthemis species, especially
- Anthemis nobilis L
- Anthemis arvensis L
- Anthemis cotula L
- Anthemis tinctoria L
- Ormenis multicaulis Br.-Bl. & Maire
- Eriocephalus punctulatus DC.,

whereby Matricaria recutita L. is especially preferred.

8. The composition according to one of 1 to 7, characterized in that the plant parts to be extracted are previously subjected to a steam distillation in order to remove the essential oils.

9. The composition according to one of 1 to 8, characterized in that the primary liquid organic extract is subjected to a molecular weight filtration in order to collect components having a molecular weight of less than 10 000 Dalton and to remove components having a molecular weight of more than 10 000 Dalton, whereby the obtained liquid extract fraction is occasionally processed into a dry powder.

10. The composition according to one of 1 to 8, characterized in that the primary liquid organic extract is subjected to a molecular weight filtration in order to collect components having a molecular weight of more than 10000 Dalton and to remove components having a molecular weight of less than 10000 Dalton, whereby the obtained liquid extract fraction is occasionally processed into a dry powder.

11. The composition according to one of 1 to 10, characterized in that it contains at least one pharmaceutical and/or dermatological acceptable auxiliary agent.

12. The composition according to one of 1 to 11, characterized in that said treatment comprises the simultaneous or sequential administration of this composition and of at least one anti-tumor agent

13. The composition according to 12, characterized in that said treatment comprises

- the administration of this composition during at least 2 days, preferably during at least 5 days in order to synchronize said abnormal proliferative mammalian cells, especially said cancer cells, and to arrest them in the S-phase, and then
- this administration is stopped at least 6 hours, preferably at least 24 hours, before the start of the administration of at least one anti-tumor agent, whereby
- during the administration of said anti-tumor agent this composition is not administered, and whereby
- during the following wash-out phase of said anti-tumor agent this composition is also not administered.

14. The composition according to 13, characterized in that said treatment cycle is repeated at least twice, preferably at least 3 times.

15. The composition according to one of 12 to 14, characterized in that said composition consists of an organic extract of at least one Chamomilla plant and/or of at least one Achillea plant and of at least one anti-tumor agent, and occasionally of at least one pharmaceutical and/or dermatological acceptable auxiliary agent.

16. The composition according to one of 12 to 15, characterized in that said anti-tumor agent is of natural, semi-synthetic or synthetic origin.

17. The composition according to one of 12 to 16, characterized in that said anti-tumor agent is selected from the group consisting of

- antimetabolites,
- DNA-alkylating agents,
- mitosis inhibitors, and
- DNA-intercalating agents.

18. The composition according to one of 1 to 17, characterized in that the primary extract contains at least one water-soluble contaminant having lipid groups which is removed by the following steps:

(i) contacting the composition with a lipophilic component that forms a complex with the contaminant;
(ii) a first removal step of removing material having a particle size larger than the complex formed in step (i); and
(iii) a second removal step of removing the complex formed in step (i).

19. The composition according to 18, characterized in that the contaminant is an endotoxin, preferably selected from the group consisting of fragments of the cell wall or fragments of molecules constituting the cell wall of Gram negative bacteria, for example selected from the group consisting of lipopolysaccharides and carbohydrates having protein groups, in particular glycoproteins having at least one lipid chain.

20. The composition according to one of 18 to 19, characterized in that the endotoxin has a molecular weight in excess of 10000 Dalton, preferably in excess of 5000 Dalton, yet more preferably in excess of 1000 Dalton and most preferably in excess of 500 Dalton.

21. The composition according to one of 18 to 20, characterized in that the lipophilic component is an oil, preferably a fatty oil.

22. The composition according to one of 18 to 21, characterized in that step (iii) comprises ultrafiltration, preferably using a filter having a pore size in the range from 0.001 to 0.02 micrometres, more preferably from 0.001 to 0.01 micrometres.

23. The composition according to one of 1 to 22, characterized in that the extract is free or essentially free of endotoxins, whereby the extract contains endotoxins in an amount of not more than 100 EU/ml - endotoxin units per ml according to European Pharmacopeia -, preferably not more than 50 EU/ml, for example not more than 25 EU/ml.

24. The composition according to one of 1 to 23, characterized in that the treatment is realized by injection of the composition or by oral, rectal or topical administration.

25. Use of a composition comprising an organic extract of at least one Chamomilla plant and/or of at least one Achillea plant according to one of 1 to 24 for the treatment of an abnormal proliferative and/or viral condition, with the proviso that for the treatment of said abnormal proliferative condition the mono/single extract of Achillea millefolium (L.) is excluded, and with the further proviso that for the treatment of said viral condition the mono/single extract of Matricaria chamomilla (L.) is excluded.

26. Use according to 25, characterized in that the composition is such a composition as defined in one of 2 to 24.

27. Use of a composition comprising an organic extract of at least one Chamomilla plant and/or of at least one Achillea plant according to one of 1 to 24 for the preparation of a medicament for the treatment of an abnormal proliferative and/or viral condition, with the proviso that for the treatment of said abnormal proliferative condition the mono/single extract of Achillea millefolium (L.) is excluded, and with the further proviso that for the treatment of said viral condition the mono/single extract of Matricaria chamomilla (L.) is excluded.

28. A method for the treatment of the human or animal body suffering under an abnormal proliferative and/or viral condition, characterized in that to the patient is applied or administered a composition containing an organic extract of at least one Chamomilla plant and/or of at least one Achillea plant according to one of 1 to 24, with the proviso that for the treatment of said abnormal proliferative condition the mono/single extract of Achillea millefolium (L.) is excluded, and with the further proviso that for the treatment of said viral condition the mono/single extract of Matricaria chamomilla (L.) is excluded.

## 5) Bibliography

**1** Toshie H., Noriko N.M., Yoshiyuki A., Mittsuhiro N., Toshiro Y., Naohito O. Granulocyte-Macrophage Colony-Stimulating Factor (GM-CSF) Regulates Cytokine Induction by 1,3-$\beta$-D-Glucan SCG in DBA/2 Mice in vitro. J. of IFN and Cytokine Research 24:478-489 (2004).

**2** Golenbock D.T., Hampton R.Y., Qureshi N., Takayama K., Raetz C. R. H. (1991) Lipid A-like molecules that antagonize the effect of endotoxins on human monocytes. J. Biol. Chem. 266 (29): 19490-498.

**3** Garrelds I.M., van Hal P. Th. W., Haakmat R. C., Hoogsteden H. C., Saxena P. R., Zijlstra F. J.: Time dependent production of cytokines and eicosanoides by human monocytic leukaemia U937 cells; effects of glucocorticosteroids. Mediators of Inflammation: 8, 229-235 (1999).

**4** Lindl T. (2000) Zell- und Gewebekultur: Einführung in die Grundlagen sowie ausgewählte Methoden und Anwendungen. 4.überarb. und erw. Auflage - Heidelberg; Berlin: Spectrum, Akad. Verlag (ISBN 3-8274-0803-2).

**5** Decker T. & Lohmann-Matthes M.L. (1988) A quick and simple method for the quantification of lactate dehydrogenase release in measurements of cellular cytotoxicity and tumor necrosis factor activity. J. Immunol Methods 15 (1): 61-69.

## Claims

1. A composition comprising an organic extract of at least one Chamomilla plant and/or of at least one Achillea plant wherein the extracts are obtainable from Flores tubiformis of the Chamomilla plant that are separated from the inflorescence and are free of apigenin and apigenin-glycosides, and/or from Flores tubiformis of the Achillea plant separated from the inflorescence, **characterized in that** the Chamomilla plant is selected from the group consisting of

   - Matricaria species, especially
   - Matricaria recutita L
   - Matricaria discoidea DC.
   - Anthemis species, especially
   - Anthemis nobilis L
   - Anthemis arvensis L
   - Anthemis cotula L
   - Anthemis tinctoria L
   - Ormenis multicaulis Br.-Bl. & Maire
   - Eriocephalus punctulatus DC.,

   and **characterized in that** said extracts are obtainable by an extraction with an alcohol or a ketone,
   with the proviso that for the treatment of an abnormal proliferative condition the mono/single extract of Achillea millefolium (L.) is excluded, and with the further proviso that for the treatment of a viral condition the mono/single extract of Matricaria chamomilla(L.) is excluded.

2. The composition according to claim 1, **characterized in that** said organic extracts are obtainable by an extraction with an alcohol with 2 to 6 carbon atoms, whereby ethanol is especially preferred, or with a ketone with 3 to 5 carbon atoms, whereby acetone is especially preferred.

3. The composition according to claim 2, **characterized in that** said organic extracts are obtainable by an extraction with ethanol as extraction solvent, wherein the concentration of ethanol is at least 80 % (m/m), preferably at least 90 % (m/m) and most preferably at least 99 % (m/m).

4. The composition according to one of claims 1 to 3, **characterized in that** the Chamomilla plant is Matricaria recutita L..

5. The composition according to one of claims 1 to 4, **characterized in that** the plant parts to be extracted are previously subjected to a steam distillation in order to remove the essential oils.

6. The composition according to one of claims 1 to 5, **characterized in that** it is suitable for oral administration or in a form suitable for oral use.

7. The composition according to one of claims 1 to 5, **characterized in that** it is suitable for rectal administration.

8. The composition according to one of claims 1 to 5, **characterized in that** it is suitable for topical administration.

9. Use of an organic extract of at least one Chamomilla plant and/or of at least one Achillea plant for the preparation of a composition comprising said extracts wherein the extracts are obtainable from Flores tubiformis of the Chamomilla plant that are separated from the inflorescence and are free of apigenin and apigenin-glycosides, and/or from Flores tubiformis of the Achillea plant separated from the inflorescence, **characterized in that** the Chamomilla plant is selected from the group consisting of

- Matricaria species, especially
- Matricaria recutita L
- Matricaria discoidea DC.
- Anthemis species, especially
- Anthemis nobilis L
- Anthemis arvensis L
- Anthemis cotula L
- Anthemis tinctoria L
- Ormenis multicaulis Br.-Bl. & Maire
- Eriocephalus punctulatus DC.,

and **characterized in that** said extracts are obtainable by an extraction with an alcohol or a ketone, with the proviso that for the treatment of an abnormal proliferative condition the mono/single extract of Achillea millefolium (L.) is excluded, and with the further proviso that for the treatment of a viral condition the mono/single extract of Matricaria chamomilla(L.) is excluded.

10. Use according to claim 9, **characterized in that** said organic extracts are obtainable by an extraction with an alcohol with 2 to 6 carbon atoms, whereby ethanol is especially preferred, or with a ketone with 3 to 5 carbon atoms, whereby acetone is especially preferred.

11. Use according to claim 10, **characterized in that** said organic extracts are obtainable by an extraction with ethanol as extraction solvent, wherein the concentration of ethanol is at least 80 % (m/m), preferably at least 90 % (m/m) and most preferably at least 99 % (m/m).

12. Use according to one of claims 9 to 11, **characterized in that** the Chamomilla plant is Matricaria recutita L..

13. Use according to one of claims 9 to 12, **characterized in that** the plant parts to be extracted are previously subjected to a steam distillation in order to remove the essential oils.

14. Use according to one of claims 9 to 13, **characterized in that** the composition is suitable for oral administration or in a form suitable for oral use.

15. Use according to one of claims 9 to 13, **characterized in that** the composition is suitable for rectal administration.

16. Use according to one of claims 9 to 13, **characterized in that** the composition is suitable for topical administration.

17. A composition for for use in a therapeutic treatment comprising an organic extract of at least one Chamomilla plant and/or of at least one Achillea plant wherein the extracts are obtainable from Flores tubiformis of the Chamomilla plant that are separated from the inflorescence and are free of apigenin and apigenin-glycosides, and/or from Flores tubiformis of the Achillea plant separated from the inflorescence, **characterized in that** the Chamomilla plant is selected from the group consisting of

- Matricaria species, especially
- Matricaria recutita L
- Matricaria discoidea DC.
- Anthemis species, especially
- Anthemis nobilis L
- Anthemis arvensis L
- Anthemis cotula L
- Anthemis tinctoria L
- Ormenis multicaulis Br.-Bl. & Maire
- Eriocephalus punctulatus DC.,

and **characterized in that** said extracts are obtainable by an extraction with an alcohol or a ketone, with the proviso that for the treatment of an abnormal proliferative condition the mono/single extract of Achillea millefolium (L.) is excluded, and with the proviso that for the treatment of a viral condition the mono/single extract of Matricaria chamomilla(L.) is excluded.

**Patentansprüche**

1. Zubereitung, einen organischen Extrakt aus mindestens einer Kamillenpflanze und/oder mindestens einer Achilleapflanze enthaltend, wobei die Extrakte aus flores tubiformis der Kamillenpflanze, die vom Blütenstand getrennt wurden und frei von Apigenin und Apigenin-Glykosiden sind, und/oder aus flores tubiformis der Achilleapflanze, die vom Blütenstand getrennt wurden, gewonnen werden können, **dadurch gekennzeichnet, dass** die Kamillenpflanze aus der Gruppe gewählt wird, bestehend aus

- Matricaria species, insbesondere
- Matricaria recutita L
- Matricaria discoidea DC.
- Anthemis species, insbesondere
- Anthemis nobilis L
- Anthemis arvensis L
- Anthemis cotula L
- Anthemis tinctoria L
- Ormenis multicaulis Br.-Bl. & Maire
- Eriocephalus punctulatus DC.,

und **dadurch gekennzeichnet, dass** die genannten Extrakte durch Extraktion mit einem Alkohol oder einem Keton gewonnen werden können, mit der Einschränkung, dass zur Behandlung eines abnorm proliferativen Leidens der alleinige Extrakt aus Achillea millefolium (L.) ausgeschlossen ist und mit der weiteren Einschränkung, dass zur Behandlung eines Virus-Leidens der alleinige Extrakt aus Matricaria chamomilla (L.) ausgeschlossen ist.

2. Zubereitung nach Patentanspruch 1, **dadurch gekennzeichnet, dass** die genannten organischen Extrakte durch Extraktion mit einem Alkohol mit 2 bis 6 Kohlenstoffatomen gewonnen werden können, wobei Ethanol besonders bevorzugt wird, oder mit einem Keton mit 3 bis 5 Kohlenstoffatomen, wobei Azeton besonders bevorzugt wird.

**3.** Zubereitung nach Patentanspruch 2, **dadurch gekennzeichnet, dass** die genannten organischen Extrakte durch Extraktion mit Ethanol als Extraktionsmittel gewonnen werden können, wobei die Ethanolkonzentration mindestens 80% (m/m) beträgt, vorzugsweise mindestens 90% (m/m) und besonders bevorzugt mindestens 99% (m/m).

**4.** Zubereitung nach einem der Patentansprüche 1 bis 3, **dadurch gekennzeichnet, dass** die Kamillenpflanze Matricaria recutita L. ist.

**5.** Zubereitung nach einem der Patentansprüche 1 bis 4, **dadurch gekennzeichnet, dass** die zu extrahierenden Pflanzenteile vorab einer Dampfdestillation unterworfen werden, um die ätherischen Öle zu entfernen.

**6.** Zubereitung nach einem der Patentansprüche 1 bis 5, **dadurch gekennzeichnet, dass** sie zur oralen Verabreichung geeignet ist oder in einer Form vorliegt, die zur oralen Verabreichung geeignet ist.

**7.** Zubereitung nach einem der Patentansprüche 1 bis 5, **dadurch gekennzeichnet, dass** sie zur rectalen Verabreichung geeignet ist.

**8.** Zubereitung nach einem der Patentansprüche 1 bis 5, **dadurch gekennzeichnet, dass** sie zur örtlichen Anwendung geeignet ist.

**9.** Gebrauch eines organischen Extraktes aus mindestens einer Kamillenpflanze und/oder mindestens einer Achilleapflanze zur Bereitung einer Zubereitung, die die genannten Extrakte enthält, wobei die Extrakte aus flores tubiformis der Kamillenpflanze, die vom Blütenstand getrennt wurden und frei von Apigenin und Apigenin-Glykosiden sind, und/oder aus flores tubiformis der Achilleapflanze, die vom Blütenstand getrennt wurden, gewonnen werden können, **dadurch gekennzeichnet, dass** die Kamillenpflanze aus der Gruppe gewählt wird, bestehend aus

- Matricaria species, insbesondere
- Matricaria recutita L
- Matricaria discoidea DC
- Anthemis species, insbesondere
- Anthemis nobilis L
- Anthemis arvensis L
- Anthemis cotula L
- Anthemis tinctoria L
- Ormenis multicaulis Br.-Bl. & Maire
- Eriocephalus punctulatus DC,

und **dadurch gekennzeichnet, dass** die genannten Extrakte durch Extraktion mit einem Alkohol oder einem Keton gewonnen werden können,
mit der Einschränkung, dass zur Behandlung eines abnorm proliferativen Leidens der alleinige Extrakt aus Achillea millefolium (L.) ausgeschlossen ist und mit der weiteren Einschränkung, dass zur Behandlung eines Virus-Leidens der alleinige Extrakt aus Matricaria chamomilla (L.) ausgeschlossen ist.

**10.** Gebrauch nach Patentanspruch 9, **dadurch gekennzeichnet, dass** die genannten organischen Extrakte durch Extraktion mit einem Alkohol mit 2 bis 6 Kohlenstoffatomen gewonnen werden können, wobei Ethanol besonders bevorzugt wird, oder mit einem Keton mit 3 bis 5 Kohlenstoffatomen, wobei Azeton besonders bevorzugt wird.

**11.** Gebrauch nach Patentanspruch 10, **dadurch gekennzeichnet, dass** die genannten organischen Extrakte durch Extraktion mit Ethanol als Extraktionsmittel gewonnen werden können, wobei die Ethanolkonzentration mindestens 80% (m/m) beträgt, vorzugsweise mindestens 90% (m/m) und besonders bevorzugt mindestens 99% (m/m).

**12.** Gebrauch nach einem der Patentansprüche 9 bis 11, **dadurch gekennzeichnet, dass** die Kamillenpflanze Matricaria recutita L. ist.

**13.** Gebrauch nach einem der Patentansprüche 9 bis 12, **dadurch gekennzeichnet, dass** die zu extrahierenden Pflanzenteile vorab einer Dampfdestillation unterworfen werden, um die ätherischen Öle zu entfernen.

**14.** Gebrauch nach einem der Patentansprüche 9 bis 13, **dadurch gekennzeichnet, dass** die Zubereitung zur oralen Verabreichung geeignet ist oder in einer Form vorliegt, die zur oralen Verabreichung geeignet ist.

15. Gebrauch nach einem der Patentansprüche 9 bis 13, **dadurch gekennzeichnet, dass** die Zubereitung zur rectalen Verabreichung geeignet ist.

16. Gebrauch nach einem der Patentansprüche 9 bis 13, **dadurch gekennzeichnet, dass** die Zubereitung zur örtlichen Anwendung geeignet ist.

17. Zubereitung zum Gebrauch in einer therapeutischen Behandlung, einen organischen Extrakt aus mindestens einer Kamillenpflanze und/oder mindestens einer Achilleapflanze enthaltend, wobei die Extrakte aus flores tubiformis der Kamillenpflanze, die vom Blütenstand getrennt wurden und frei von Apigenin und Apigenin-Glykosiden sind, und/oder aus flores tubiformis der Achilleapflanze, die vom Blütenstand getrennt wurden, gewonnen werden können, **dadurch gekennzeichnet, dass** die Kamillenpflanze aus der Gruppe gewählt wird, bestehend aus

- Matricaria species, insbesondere
- Matricaria recutita L
- Matricaria discoidea DC.
- Anthemis species, insbesondere
- Anthemis nobilis L
- Anthemis arvensis L
- Anthemis cotula L
- Anthemis tinctoria L
- Ormenis multicaulis Br.-Bl. & Maire
- Eriocephalus punctulatus DC.,

und **dadurch gekennzeichnet, dass** die genannten Extrakte durch Extraktion mit einem Alkohol oder einem Keton gewonnen werden können,
mit der Einschränkung, dass zur Behandlung eines abnorm proliferativen Leidens der alleinige Extrakt aus Achillea millefolium (L.) ausgeschlossen ist und mit der weiteren Einschränkung, dass zur Behandlung eines Virus-Leidens der alleinige Extrakt aus Matricaria chamomilla (L.) ausgeschlossen ist.

## Revendications

1. Composition comprenant un extrait organique d'au moins une plante de camomille et/ou d'au moins une plante d'Achillea, les extraits pouvant être obtenus à partir des fleurs en tube de la plante de camomille qui sont séparées des inflorescences et qui sont exemptes d'apigénine et de glycosides d'apigénine et/ou des fleurs en tube d'Achillea qui sont séparées des inflorescences, **caractérisée en ce que** la plante de camomille est sélectionnée dans le groupe comportant :

- l'espèce camomille matricaire en particulier
- la camomille Matricaria recutita L,
- la camomille Matricaria discoidea DC,
- l'espèce Anthemis en particulier,
- Anthemis nobilis L,
- Anthemis arvensis L,
- Anthemis cotula L,
- Anthemis tinctoria L,
- Ormenis multicaulis Br et Bl & Maire
- Erlocephalus punctulatus DC

et **caractérisée en ce que** lesdits extraits peuvent être obtenus par l'extraction avec un alcool ou un cétone, à condition que pour le traitement de l'état prolifératif anormal l'extrait mono/unique d'Achiliea millefolium (L.) est exclu et avec la condition supplémentaire que pour le traitement d'un état viral l'extrait mono/unique de Matricaria chamomilla (L.) est exclu.

2. Composition selon la revendication 1, **caractérisée en ce que** lesdits extraits organiques peuvent être obtenus par l'extraction avec un alcool avec 2 à 6 atomes de carbones, l'éthanol étant préféré en particulier ou avec un cétone avec 3 à 5 atomes de carbone, l'acétone étant préféré en particulier.

**3.** Composition selon la revendication 2, **caractérisée en ce que** lesdits extraits organiques peuvent être obtenus par l'extraction avec de l'éthanol comme solvant d'extraction, la concentration d'éthanol étant d'au moins 80% (m/m), de préférence de 90% (m/m) et de manière encore plus préférée d'au moins 99% (m/m).

**4.** Composition selon l'une des revendications 1 à 3, **caractérisée en ce que** la plante de camomille est Matricaria recutita L.

**5.** Composition selon l'une des revendications 1 à 4, **caractérisée en ce que** les parties de plantes à extraire sont auparavant soumises à une distillation à la vapeur pour retirer les huiles essentielles.

**6.** Composition selon l'une des revendications 1 à 5, **caractérisée en ce qu'**elle convient pour une administration orale ou dans une forme appropriée pour l'utilisation orale.

**7.** Composition selon l'une des revendications 1 à 5, **caractérisée en ce qu'**elle convient pour une administration rectale.

**8.** Composition selon l'une des revendications 1 à 5, **caractérisée en ce qu'**elle convient pour une administration locale.

**9.** Utilisation d'un extrait organique d'au moins une plante de camomille et/ou d'au moins une plante d'Achillea pour la préparation d'une composition qui comprend lesdits extraits, les extraits pouvant être obtenus à partir des fleurs en tube de la plante de camomille qui sont séparées des inflorescences et qui sont exemptes d'apigénine et de glycosides d'apigénine et/ou des fleurs en tube de la plante d'Achillea séparées des inflorescences, **caractérisée en ce que** la plante de camomille est sélectionnée dans le groupe comportant :

- l'espèce camomille matricaire en particulier
- la camomille Matricaria recutita L,
- la camomille Matricaria discoidea DC,
- l'espèce Anthemis en particulier,
- Anthémis nobilis L,
- Anthemis arvensis L,
- Anthemis cotula L,
- Anthémis tinctoria L,
- Ormenis multicaulis Br et Bl & Maire
- Erlocephalus punctulatus DC.

et **caractérisée en ce que** lesdits extraits peuvent être obtenus par l'extraction avec un alcool ou un cétone, à condition que pour le traitement de l'état prolifératif anormal l'extrait mono/unique d'Achillea millefolium (L.) est exclu et avec la conditions supplémentaire que pour le traitement d'un état viral l'extrait mono/unique de Matricaria chamomilla (L.) est exclu.

**10.** Utilisation selon la revendication 9, **caractérisée en ce que** lesdits extraits organiques peuvent être obtenus par l'extraction avec un alcool avec 2 à 6 atomes de carbones, l'éthanol étant préféré en particulier ou avec un cétone avec 3 à 5 atomes de carbone, l'acétone étant préféré en particulier.

**11.** Utilisation selon la revendication 10, **caractérisée en ce que** lesdits extraits organiques peuvent être obtenus par l'extraction avec de l'éthanol comme solvant d'extraction, la concentration d'éthanol étant d'au moins 80% (m/m), de préférence de 90% (m/m) et de manière encore plus préférée d'au moins 99% (m/m).

**12.** Utilisation selon l'une des revendications 9 à 11, **caractérisée en ce que** la plante de camomille est Matricaria recutita L.

**13.** Utilisation selon l'une des revendications 9 à 12, **caractérisée en ce que** les parties de plantes à extraire sont auparavant soumises à une distillation à la vapeur pour retirer les huiles essentielles.

**14.** Utilisation selon l'une des revendications 9 à 13, **caractérisée en ce que** la composition convient pour une administration orale ou dans une forme appropriée pour l'utilisation orale.

**15.** Utilisation selon l'une des revendications 9 à 13, **caractérisée en ce que** la composition convient pour une administration rectale.

16. Utilisation selon l'une des revendications 9 à 13, **caractérisée en ce que** la composition convient pour une administration locale.

17. Composition pour utilisation dans un traitement thérapeutique comprenant un extrait organique d'au moins une plante de camomille et/ou d'au moins une plante d'Achillea, les extraits pouvant être obtenus à partir des fleurs en tube de la plante de camomille qui sont séparées des inflorescences et qui sont exemptes d'apigénine et de glycosides d'apigénine et/ou des fleurs en tube de la plante d'Achillea séparées des inflorescences, **caractérisée en ce que** la plante de camomille est sélectionnée dans le groupe comportant :

- l'espèce camomille matricaire en particulier
- la camomille Matricaria recutita L,
- la camomille Matricaria discoidea DC,
- l'espèce Anthemis en particulier,
- Anthemis nobilis L,
- Anthemis arvensis L,
- Anthemis cotula L,
- Anthemis tinctoria L,
- Ormenis multicaulis Br et Bl & Maire
- Erlocephalus punctulatus DC.

et **caractérisée en ce que** lesdits extraits peuvent être obtenus par l'extraction avec un alcool ou un cétone, à condition que pour le traitement de l'état prolifératif anormale l'extrait mono/nique d'Achillea millefolium (L.) est exclu et avec la conditions supplémentaire que pour le traitement d'un état viral l'extrait mono/unique de Matricaria chamomilla (L.) est exclu.

Fig. 1

Fig. 2

Fig. 3

**Fig. 4**

Fig. 5

**Fig. 6**

**Fig. 7**

**Fig. 8**

**Fig. 9**

| DNA: | 1 | 2 | 3 | 4 | 5 | 6 | 7 | 8 | 9 |
|---|---|---|---|---|---|---|---|---|---|
| Camptothecin [µM]: | - | - | 100 | 500 | - | - | - | - | - |
| Example 7 [µg/ml]: | - | - | - | - | 0.3 | 1 | 3 | 10 | 30 |
| Topoisomerase I: | - | + | + | + | + | + | + | + | + |

**Fig. 10**

| DNA: | 1 | 2 | 3 | 4 | 5 | 6 | 7 | 8 | 9 | 10 | 11 | 12 |
|---|---|---|---|---|---|---|---|---|---|---|---|---|
| Etoposide: | - | - | + | - | - | - | - | - | - | - | - | - |
| Example 7 [µg/ml]: | - | - | - | 0.3 | 1 | 3 | - | - | - | - | - | - |
| Example 8 [µg/ml]: | - | - | - | - | - | - | 0.3 | 1 | 3 | - | - | - |
| Example 9 [µg/ml]: | - | - | - | - | - | - | - | - | - | 0.3 | 1 | 3 |
| Topoisomerase II: | - | + | + | + | + | + | + | + | + | + | + | + |

**Fig. 11**

**Fig. 12**

| DNA: | 1 | 2 | 3 | 4 | 5 | 6 | 7 | 8 | 9 | 10 | 11 | 12 | 13 | 14 |

| | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| Etoposide [1000 µM]: | - | - | + | - | - | - | - | - | - | - | - | - | - | - |
| Example 7 [µg/ml]: | - | - | - | 0.3 | 1 | 3 | 10 | 30 | - | - | - | - | - | - |
| α-Bisabolol [µg/ml]: | - | - | - | - | - | - | - | - | 0.3 | 3 | 30 | - | - | - |
| Essential oil [µg/ml]: | - | - | - | - | - | - | - | - | - | - | - | 0.3 | 3 | 30 |
| Topoisomerase II: | - | + | + | + | + | + | + | + | + | + | + | + | + | + |

**Fig. 13**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03101479 A1
- WO 2007057651 A1
- WO 0113929 A1
- WO 2008146009 A1
- US 6300370 B1

### Non-patent literature cited in the description

- **P. ČERNAJ et al.** *Biologia Plantarum,* May 1983, vol. 25 (3), 221-224
- **R. D. LOGGIA et al.** *Pharmacological Research Communications,* 1982, vol. 14 (2), 153-162
- **P.VILAGINES ; P. DELAVEAU ; R. VILAGINES.** Inhibition de la replication du poliovirus par un extrait de Matricaria chamomilla (L.). *Comptes Rendus de l'Academie des Sciences,* 1985, vol. 301 (6), 289-294
- **GRATZNER, H. G.** *Science,* 1982, vol. 218, 474-475
- **TOSHIE H. ; NORIKO N.M. ; YOSHIYUKI A. ; MITTSUHIRO N. ; TOSHIRO Y. ; NAOHITO O.** Granulocyte-Macrophage Colony-Stimulating Factor (GM-CSF) Regulates Cytokine Induction by 1,3-β-D-Glucan SCG in DBA/2 Mice in vitro. *J. of IFN and Cytokine Research,* 2004, vol. 24, 478-489
- **GOLENBOCK D.T. ; HAMPTON R.Y. ; QURESHI N. ; TAKAYAMA K. ; RAETZ C. R. H.** Lipid A-like molecules that antagonize the effect of endotoxins on human monocytes. *J. Biol. Chem.,* 1991, vol. 266 (29), 19490-498
- **GARRELDS I.M. ; VAN HAL P. TH. W. ; HAAKMAT R. C. ; HOOGSTEDEN H. C. ; SAXENA P. R. ; ZIJLSTRA F. J.** Time dependent production of cytokines and eicosanoides by human monocytic leukaemia U937 cells; effects of glucocorticosteroids. *Mediators of Inflammation,* 1999, vol. 8, 229-235
- **LINDL T.** Zell- und Gewebekultur: Einführung in die Grundlagen sowie ausgewählte Methoden und Anwendungen. Akad. Verlag, 2000
- **DECKER T. ; LOHMANN-MATTHES M.L.** A quick and simple method for the quantification of lactate dehydrogenase release in measurements of cellular cytotoxicity and tumor necrosis factor activity. *J. Immunol Methods,* 1988, vol. 15 (1), 61-69